(19) Europäisches Patentamt
European Patent Office
Office européen des brevets

(11) Veröffentlichungsnummer: **0 007 991 A1**

(12) **EUROPÄISCHE PATENTANMELDUNG**

(21) Anmeldenummer: **79102093.6**

(22) Anmeldetag: **25.06.79**

(51) Int. Cl.³: **C 07 D 233/50,** A 61 K 31/415

(30) Priorität: **22.07.78 DE 2832310**

(43) Veröffentlichungstag der Anmeldung: **20.02.80 Patentblatt 80/4**

(84) Benannte Vertragsstaaten: **AT BE CH DE FR GB IT LU NL SE**

(71) Anmelder: **C.H. BOEHRINGER SOHN, Postfach 200, D-6507 Ingelheim am Rhein (DE)**

(72) Erfinder: **Stähle, Helmut, Dr., Rotweinstrasse 23, D-6507 Ingelheim (DE)**
Erfinder: **Köppe, Herbert, Dr., Neuweg 72, D-6507 Ingelheim (DE)**
Erfinder: **Kummer, Werner, Dr., Georg-Scheuing-Strasse 15, D-6507 Ingelheim (DE)**
Erfinder: **Kobinger, Walter, Prof. Dr., Belghofergasse 27, A-1120 Wien (AT)**
Erfinder: **Lillie, Christian, Dr.Mag., Hansi-Niese-Weg 12, A-1130Wien (AT)**
Erfinder: **Pichler, Ludwig, Dr., Gusshausstrasse 24, A-1040 Wien (AT)**

(54) **Neue substituierte 2-Phenylamino-imidazoline-(2), deren Säureadditionssalze, diese enthaltende Arzneimittel und Verfahren zur Herstellung derselben.**

(57) Die Erfindung betrifft substituierte 2-Phenylamino-imidazoline-(2) der allgemeinen Formel I

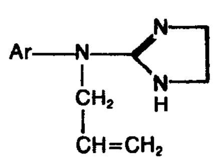

I

worin Ar den Rest 2,6-Dibromphenyl, 2-Fluor-6-trifluormethylphenyl, 2-Fluor-6-chlorphenyl, 2-Chlor-6-bromphenyl, 2,6-Difluorphenyl, 2,4-Dichlorphenyl, 2-Brom-3-chlorphenyl, 2,6-Dichlor-4-hydroxy-carbonyl-phenyl, 2,6-Dichlor-4-äthoxycarbonyl-phenyl, 2,6-Dichlor-4-methoxyphenyl oder 2-Brom-6-fluorphenyl bedeutet.

Die Verbindungen sind als Herz- bzw. Coronartherapeutika verwendbar.

**EP 0 007 991 A1**

Die Erfindung betrifft neue substituierte 2-Phenylamino-imidazoline-(2) der allgemeinen Formel

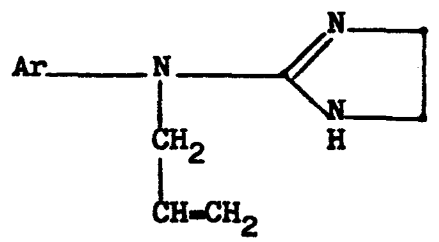

I

sowie deren physiologisch verträgliche Säureadditionssalze mit wertvollen therapeutischen Eigenschaften.

In der Formel I bedeutet Ar den Rest 2,6-Dibromphenyl, 2-Fluor-6-trifluormethylphenyl, 2-Fluor-6-chlorphenyl, 2-Chlor-6-bromphenyl, 2,6-Difluorphenyl, 2,4-Dichlorphenyl, 2-Brom-3-chlorphenyl, 2,6-Dichlor-4-hydroxycarbonyl-phenyl, 2,6-Dichlor-4-äthoxycarbonyl-phenyl, 2,6-Dichlor-4-methoxy-phenyl oder 2-Brom-6-fluorphenyl.

Die Herstellung der Verbindungen der Formel I erfolgt durch:

a) Umsetzung eines 2-Phenylimino-imidazolidins der allgemeinen Formel

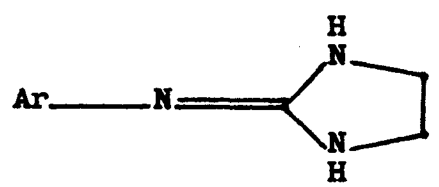

II

in der Ar die oben genannten Bedeutungen besitzt, mit einem Halogenid der allgemeinen Formel

$Hal-CH_2-CH=CH_2$ III

worin Hal ein Chlor-, Brom- oder Jodatom bedeutet; oder

b) Umsetzung einer Verbindung der allgemeinen Formel

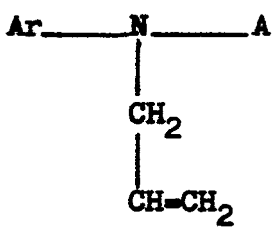

IV

in der Ar wie oben angegeben definiert ist und A eine Cyanogruppe oder den Rest 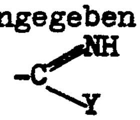 bedeutet, wobei Y eine Alkoxy- oder Alkylthiogruppe mit bis zu 4 C-Atomen oder eine Sulfhydryl- oder Aminogruppe darstellt, mit Äthylendiamin oder dessen Säureadditionssalzen.

Bei der Alkylierung der 2-Arylimino-imidazolidine der Formel II nach Verfahren a) erfolgt die Substitution ausschließlich am Brückenstickstoffatom. Bei der Umsetzung nach den Verfahren b) ist die Konstitution der Endverbindungen durch die Synthese festgelegt. Die Position der Substituenten läßt sich außer durch die Synthese auch durch die NMR-Spektroskopie festlegen. (Vgl. H. Stähle und K.-H. Pook, Liebigs Ann. Chem. 751, 159 ff (1971).

Die Umsetzung nach Verfahren a) erfolgt zweckmäßigerweise durch Erhitzen der Reaktionspartner - vorzugsweise in Gegenwart eines polaren oder unpolaren organischen Lösungsmittels - auf Temperaturen von etwa 50 - 150°C. Die speziellen Reaktionsbedingungen

hängen in starkem Maße von der Reaktivität der Umsetzungsteilnehmer ab. Es empfiehlt sich, bei der Alkylierung das Halogenid im Überschuß anzuwenden und die Umsetzung in Gegenwart eines säurebindenden Mittels durchführen.

Bei dem Verfahren nach b) ist es erforderlich, bei erhöhter Temperatur, zwischen 60°C und 180°C zu arbeiten. Lösungsmittel sind nicht erforderlich. Es ist zweckmäßig, das als Reaktionspartner verwendete Äthylendiamin bzw. dessen Säureadditionssalz, im Überschuß anzuwenden.

Ausgangsverbindungen der Formel II sind z. B. in den belgischen Patenten Nr. 623 305, 687 657 und 705 944 beschrieben.

Verbindungen der Formel IV erhält man ausgehend von Anilinen, durch Umsetzung mit Verbindungen der Formel III und nachfolgende Reaktion der dabei entstehenden sekundären Amine, mit Cyanaten oder Thiocyanaten wobei Harnstoffe, bzw. Thioharnstoffe gebildet werden. Harnstoffe und Thioharnstoffe können dann weiter mit Alkylierungsmitteln in entsprechende Isouroniumsalze bzw. Isothiouroniumsalze überführt werden. Aus diesen Säureadditionsverbindungen lassen sich mit Basen die entsprechenden Isoharnstoffe bzw. Isothioharnstoffe gewinnen. Durch Wasserabspaltung aus Harnstoffen bzw. $H_2S$-Abspaltung aus Thioharnstoffen mittels Blei- oder Quecksilbersalzen, gelangt man zu Cyanamiden, an die Ammoniak unter Bildung von Guanidinen angelagert werden kann.

Die erfindungsgemäßen 2-Phenylamino-imidazoline-(2) der allgemeinen Formel I können auf übliche Weise in ihre physiologisch verträglichen Säureadditionssalze überführt werden. Zur Salzbildung geeignete Säuren sind beispielsweise Salzsäure, Bromwasserstoffsäure, Jodwasserstoffsäure, Fluorwasserstoffsäure, Schwefelsäure, Phosphorsäure, Salpetersäure, Essigsäure, Propionsäure, Buttersäure, Capronsäure, Valeriansäure, Oxalsäure, Malonsäure, Bernsteinsäure, Maleinsäure, Fumarsäure, Milchsäure, Weinsäure, Zitronensäure, Äpfelsäure, Benzoesäure, p-Hydroxybenzoesäure, p-Aminobenzoesäure, Phthalsäure, Zimt-

0007991



säure, Salicylsäure, Ascorbinsäure, Methansulfonsäure, 8-Chlortheophyllin und dergleichen.

Die neuen Verbindungen der allgemeinen Formel I und deren Säureadditionssalze wirken sehr stark bradycard und sind daher zur Therapie von Coronarerkrankungen geeignet. Die Beeinflussung der Herzfrequenz wurde an Kaninchen und an Spinalratten sowie an intakten narkotisierten Ratten untersucht.
Die Dosierung liegt bei 0,1 bis 80 mg, vorzugsweise 1 bis 30 mg.

Die Verbindungen der Formel I bzw. ihre Säureadditionssalze können auch mit andersartigen Wirkstoffen zum Einsatz gelangen. Geeignete galenische Darreichungsformen sind beispielsweise Tabletten, Kapseln, Zäpfchen, Lösungen oder Pulver; hierbei können zu deren Herstellung die üblicherweise verwendeten galenischen Hilfs-, Träger-, Spreng- oder Schmiermittel oder Substanzen zur Erzielung einer Depotwirkung Anwendung finden.

Die folgenden Beispiele erläutern die Erfindung, ohne sie zu beschränken.

**Beispiel 1**

**3,5-Dichlor-4-[N-(allyl)-N-(2-imidazolinyl-(2)-)amino]-benzoesäure**

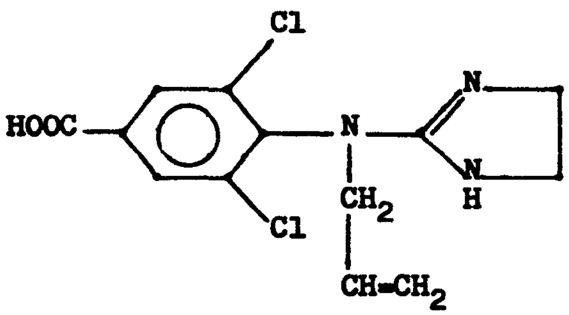

0,7 g (0,002045 Mol) 3,5-Dichlor-4-[N-(allyl)-N-(2-imidazolinyl-(2)-)amino]-benzoesäureäthylester werden zusammen mit 0,4 g (0,00716 Mol) Kaliumhydroxyd, 0,5 ml Wasser und 1 ml Äthanol kurze Zeit auf 60°C erhitzt. Hierauf wird mit Wasser verdünnt, das Reaktionsgemisch im Vakuum vom Alkohol befreit und die Lösung nach Filtration mit Eisessig auf pH 5 gepuffert. Die ausgefallene weiße Substanz wird abgesaugt mit Wasser gewaschen und getrocknet. Durch Aussalzen der Mutterlauge mit Kochsalz können weitere Substanzmengen gewonnen werden.
Ausbeute: 0,42 g (65,4 % der Theorie), Schmelzpunkt: 321-323°C
RF: 0,3 System: Essigester 70, Isopropanol 50, konz. $NH_4OH$ 20
Träger: Kieselgel G + Leuchtpigment, Detektor: UV und Kaliumjodplatinat

$C_{13}H_{13}Cl_2N_3O_2$ (314,17)

| | C | H | Cl | N |
|---|---|---|---|---|
| Ber.: | 49,70 % | 4,17 % | 22,57 % | 13,38 % |
| Gef.: | 49,50 % | 4,35 % | 22,36 % | 13,40 % |

Beispiel 2

2-[N-(Allyl)-N-(2,6-dibromphenyl)-amino]-2-imidazolin

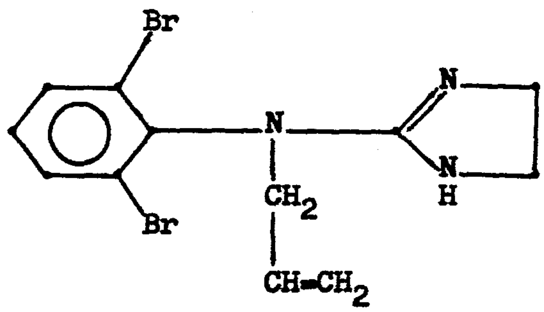

6,38 g (0,02 Mol) 2-(2,6-Dibromphenylimino)-imidazolidin werden zusammen mit 10 ml Allylbromid in 25 ml Methanol 60 Stunden lang im Rohr auf 100°C erhitzt. Nach dem Abkühlen liegen zwei Phasen vor. Die untere Phase (ölige Masse) wird zur weiteren Aufarbeitung in Wasser gelöst. Die Lösung wird sodann bei aufsteigenden pH-Werten (stufenweises Alkalisieren mit 2 N Natronlauge) fraktioniert mit Äther extrahiert. Die dünnschichtchromatografisch einheitlichen Ätherextrakte werden vereinigt, über $MgSO_4$ getrocknet, abfiltriert und im Vakuum eingeengt. Hierbei fällt zunächst ein Öl an, welches in kurzer Zeit durchkristallisiert (weiße Kristalle). Ausbeute: 1,6 g (22,3 % der Theorie), Schmelzpunkt: 134-136°C RF: 0,5 , System: Benzol 50, Dioxan 40, Äthanol 5, konz. $NH_4OH$ 5 Träger: Kieselgel G + Leuchtpigment, Detektor: UV und Kaliumjodplatinat

$C_{12}H_{13}Br_2N_3$ (359,08)

| | C | H | Br | N |
|---|---|---|---|---|
| Ber.: | 40,14 % | 3,65 % | 44,51 % | 11,70 % |
| Gef.: | 40,26 % | 3,60 % | 44,25 % | 11,49 % |

Analog den vorstehenden Beispielen wurden die folgenden Verbindungen hergestellt. Die Schmelzpunkte beziehen sich auf die Basen der Formel I. Anderenfalls ist die Salzform angegeben.

| Beispiel Nr. | Ar | Fp. °C | Ausbeute % der Theorie |
|---|---|---|---|
| 3 | 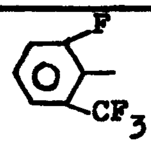 | 118-119 | 53,0 |
| 4 | 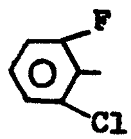 | 105-106 | 52,8 |
| 5 | 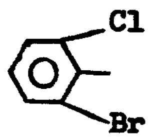 | 132-134 | 49,1 |
| 6 | 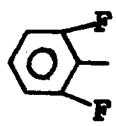 | 153-155 (Hydrobromid) | 70,2 |
| 7 | 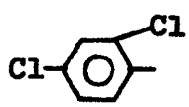 | 104 | 69,1 |
| 8 | 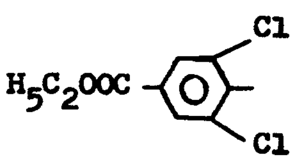 | 109-111 | 68,2 |
| 9 | 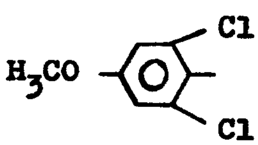 | 135 | 75,2 |
| 10 | 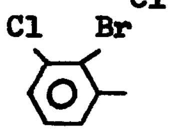 | 86-87 | 18,7 |
| 11 | 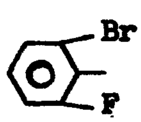 | 105-106 | 48,5 |

Formulierungsbeispiele

Beispiel A: Dragées

| | |
|---|---|
| Wirkstoff gemäß Erfindung | 5 mg |
| Milchzucker | 65 mg |
| Maisstärke | 130 mg |
| sek. Calciumphosphat | 40 mg |
| lösliche Stärke | 3 mg |
| Magnesiumstearat | 3 mg |
| kolloidale Kieselsäure | 4 mg |
| insgesamt | 250 mg |

Herstellung:
Der Wirkstoff wird mit einem Teil der Hilfsstoffe vermischt, intensiv mit einer wäßrigen Lösung der löslichen Stärke durchgeknetet und in üblicher Weise mit Hilfe eines Siebes granuliert. Das Granulat wird mit dem Rest der Hilfsstoffe vermischt und zu Dragéekernen von 250 mg Gewicht verpreßt, die dann in üblicher Weise mit Hilfe von Zucker, Talkum und Gummi arabicum dragiert werden.

Beispiel B: Ampullen

| | | |
|---|---|---|
| Wirkstoff gemäß Erfindung | | 1,0 mg |
| Natriumchlorid | | 18,0 mg |
| dest. Wasser | ad | 2,0 ml |

Herstellung:
Wirkstoff und Natriumchlorid werden in Wasser gelöst und unter Stickstoff in Glasampullen abgefüllt.

Beispiel C: Tropfen

| | | |
|---|---|---|
| Wirkstoff gemäß Erfindung | | 0,02 g |
| p-Hydroxybenzoesäuremethylester | | 0,07 g |
| p-Hydroxybenzoesäurepropylester | | 0,03 g |
| entmineralisiertes Wasser | ad | 100 ml |

PATENTANSPRÜCHE:

1. Substituierte 2-Phenylamino-imidazoline-(2) der allgemeinen Formel

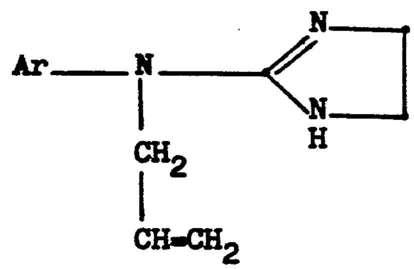

I

Ar den Rest 2,6-Dibromphenyl, 2-Fluor-6-trifluormethylphenyl, 2-Fluor-6-chlorphenyl, 2-Chlor-6-bromphenyl, 2,6-Difluorphenyl, 2,4-Dichlorphenyl, 2-Brom-3-chlorphenyl, 2,6-Dichlor-4-hydroxycarbonyl-phenyl, 2,6-Dichlor-4-äthoxycarbonyl-phenyl, 2,6-Dichlor-4-methoxyphenyl oder 2-Brom-6-fluorphenyl bedeutet sowie deren Säureadditionssalze.

2. Verfahren zur Herstellung von substituierten 2-Phenylaminoimidazolinen-(2) der allgemeinen Formel I nach Anspruch 1 und von deren Säureadditionssalzen, dadurch gekennzeichnet, daß man

a) ein 2-Phenyl-iminoimidazolidin der allgemeinen Formel

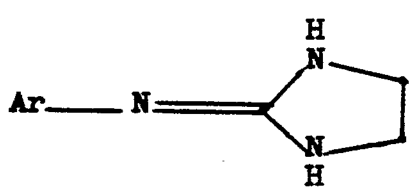

II

in der Ar die oben genannten Bedeutungen besitzt, mit einem Allylhalogenid der allgemeinen Formel

$$Hal-CH_2-CH=CH_2 \qquad III$$

worin Hal ein Chlor-, Brom- oder Jodatom bedeutet umsetzt; oder

b) eine Verbindung der allgemeinen Formel

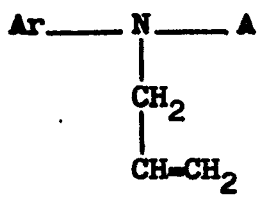

IV

in der Ar wie oben angegeben definiert ist und A eine Cyanogruppe oder den Rest 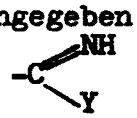 bedeutet, wobei Y eine Alkoxy- oder Alkylthiogruppe mit bis zu 4 Kohlenstoffatomen oder eine Sulfhydryl- oder Aminogruppe darstellt, mit Äthylendiamin oder dessen Säureaddtionssalzen umsetzt, und daß man gegebenenfalls das nach einem der Verfahren erhaltene Endprodukt in ein Säureadditionssalze überführt.

3. Verfahren nach Anspruch 2, dadurch gekennzeichnet, daß man die Umsetzung bei Temperaturen von etwa 50 bis 100°C durchführt.

4. Verfahren nach Anspruch 2 a) und/oder 3, dadurch gekennzeichnet, daß man die Umsetzung in Gegenwart eines polaren oder unpolaren organischen Lösungsmittels durchführt.

5. Verfahren nach Anspruch 2 a) bis 4, dadurch gekennzeichnet, daß man die Umsetzung in Gegenwart eines säurebindenden Mittels durchführt.

6. Pharmazeutische Zubereitungen, dadurch gekennzeichnet, daß sie als Wirkstoff eine oder mehrere Verbindungen der allgemeinen Formel I oder deren Säureadditionssalze enthalten.

7. Verfahren zur Herstellung von Arzneimitteln nach Anspruch 6, dadurch gekennzeichnet, daß man eine oder mehrere Verbindungen der allgemeinen Formel I oder deren Säureadditionssalze mit üblichen galenischen Hilfs-, Träger-, Spreng- oder Schmiermitteln bzw. Substanzen zur Erzielung einer Depotwirkung zu Tabletten, Kapseln, Zäpfchen, Lösungen oder Pulver formuliert.

8. Verwendung von Verbindungen der allgemeinen Formel I oder deren physiologisch verträglichen Säureadditionssalzen bei der Bekämpfung von Herz- und Coronarerkrankungen.

Europäisches Patentamt

# EUROPÄISCHER RECHERCHENBERICHT

Nummer der Anmeldung

EP 79 102 093.6

| EINSCHLÄGIGE DOKUMENTE | | | KLASSIFIKATION DER ANMELDUNG (Int.Cl.3) |
|---|---|---|---|
| Kategorie | Kennzeichnung des Dokuments mit Angabe, soweit erforderlich, der maßgeblichen Teile | betrifft Anspruch | |
| | DE - A - 1 958 201 (C.H. BOEHRINGER SOHN)<br>* Ansprüche 1, 2, 4, 5, 7, 8, 10 bis 12 *<br>-- | 1-7 | C 07 D 233/50<br>A 61 K 31/415 |
| A | DE - A1 - 2 523 103 (C.H. BOEHRINGER SOHN)<br>---- | | |

RECHERCHIERTE SACHGEBIETE (Int. Cl.3)

A 61 K 31/415
C 07 D 233/50

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| Berlin | 07-11-1979 | FROELICH |